# EUROPEAN PATENT SPECIFICATION

(11) **EP 3 344 252 B1**
(45) Date of publication and mention of the grant of the patent: **26.02.2020**
(21) Application number: 16774459.8
(22) Date of filing: 05.09.2016
(51) Int. Cl.: A61K 31/51, A61P 35/00

(54) **TREATMENT OF BILIARY DUCT CANCER**
BEHANDLUNG VON GALLENGANGKREBS
TRAITEMENT DU CANCER DES VOIES BILIAIRES

(30) Priority: 04.09.2015 GB 201515716; 04.09.2015 GB 201515712; 04.09.2015 GB 201515714; 04.09.2015 GB 201515718; 11.09.2015 US 201562217332 P; 11.09.2015 US 201562217346 P; 01.04.2016 GB 201605583; 17.05.2016 GB 201608660
(43) Date of publication of application: 11.07.2018
(73) Proprietor: Aslan Pharmaceuticals PTE Limited, Singapore 239920 (SG)
(72) Inventor: LINDMARK, Bertil, 089824 (SG); MCHALE, Mark Thomas, 089824 (SG); OOI, Lisa, 089824 (SG)
(74) Representative: Sterling IP
(86) International application number: PCT/EP2016/070890
(87) International publication number: WO 2017/037299

(56) References cited:
- WO-A1-2005/016346
- ANDERSON DEBORAH ET AL: "In vivo activity of ARRY-334543, a potent, small molecule inhibitor of EGFR/ErbB2 in combination with trastuzumab or docetaxel", PROCEEDINGS OF THE AMERICAN ASSOCIATION FOR CANCER RESEARCH ANNUAL MEETING Cancer Research, 18 April 2009 (2009-04-18), 22 April 2009 (2009-04-22), XP002764674, & 100TH ANNUAL MEETING OF THE AMERICAN-ASSOCIATION-FOR-CANCER-RESEARCH; DENVER, CA, USA; APRIL 18 -22, 2009 ISSN: 0197-016X Retrieved from the Internet: URL:http://cancerres.aacrjournals.org/cont ent/69/9_Supplement/1757 [retrieved on 2016-11-25]
- ELLARD SUSAN L ET AL: "Abstract #3603:ARRY-334543 in ErbB2 positive metastatic breast cancer and other ErbB expressing-cancers: experience from expansion cohorts on a phase I study", PROCEEDINGS OF THE AMERICAN ASSOCIATION FOR CANCER RESEARCH ANNUAL MEETING Cancer Research, 18 April 2009 (2009-04-18), 22 April 2009 (2009-04-22), XP002764103, & 100TH ANNUAL MEETING OF THE AMERICAN-ASSOCIATION-FOR-CANCER-RESEARCH; DENVER, CA, USA; APRIL 18 -22, 2009 ISSN: 0197-016X Retrieved from the Internet: URL:http://cancerres.aacrjournals.org/cont ent/69/9_Supplement/3603 [retrieved on 2016-11-07]
- KIM J. ET AL.: "664P:Phase IIa study to evaluate the biological activity of ASLAN001 in HER-1/2 co-expressing or HER-2 amplified advanced gastric cancer", ANNALS OF ONCOLOGY, vol. 25, no. Suppl 4, 2014, page iv226, XP002764105, DOI: 10.1093/annonc/mdu334.49
- Anonymous: "ASLAN Pharmaceuticals receives Orphan Drug Designation from the FDA for ASLAN001 (varlitinib) in cholangiocarcinoma", Internet , 13 August 2015 (2015-08-13), XP002764675, Retrieved from the Internet: URL:http://www.aslanpharma.com/app/uploads /2015/08/Press-Release-ASLAN001-orphan-sta tus.pdf [retrieved on 2016-11-28]
- NEHLS O ET AL: "Capecitabine plus oxaliplatin as first-line treatment in patients with advanced biliary system adenocarcinoma: a prospective multicentre phase II trial", BRITISH JOURNAL OF CANCER, vol. 98, no. 2, January 2008 (2008-01), pages 309-315, XP002764676, ISSN: 0007-0920

## Description

The present disclosure relates to a therapy, for example a monotherapy or combination therapy comprising a type I tyrosine kinase inhibitor for the treatment of biliary cancer, such as cholangiocarcinoma.

### BACKGROUND

There are many cancers that are difficult to treat and although therapy is available, there appears to exist or to come into existence, a degree of resistance to the therapy. Primary resistance may occur in that cancer does not responsive to treatment from the outset. Secondary or acquired resistance also occurs quite frequently, which means that a therapy to which the patient seems to respond, at a certain time, loses its efficacy.

There are numerous reasons for resistance, for example some cancers are discovered at a late stage and/or a simply not responsive to treatment.

Mechanisms by which cancers avoid the therapeutic effect of therapy include but are not limited to:
i) mutations which render the cancer less vulnerable to the treatment (eg mutation of the site of action of the therapy),
ii) active transportation of the drug out of the tumor, for example by p-glycolation,
iii) building up physical defences, for example stroma which inhibit certain immune responses, and
iv) certain cancers develop paths to repair damage caused by some anti-cancer therapies.

Tumor heterogeneity may also contribute to resistance, where small subpopulations of cells may acquire or stochastically already possess some of the features enabling them to emerge under selective drug pressure. This is a problem that many patients with cancer encounter, and it obviously limits the therapeutic alternatives that are effective and worsens the prognosis.

Cancer therapy guidelines describe the sequence of therapies, which are recommended and in which sequence, so that if a patients show disease progression on the first therapy ("first line"), then a next therapy ("second line") is recommended, and so on. These therapy recommendations are based on available scientific data and experience, and illustrate that resistance to one therapy does not exclude that another therapy may be effective and prolong life or shrink tumor. At a late stage cancers do not respond and no more avenues of therapy exist, and are thus judged as completely therapy refractory, unless new therapies can be found which are effective.

Cholangiocarcinoma is a prime example of both primary and secondary resistance and is considered to be an incurable and a rapidly lethal malignancy unless both the primary tumor and any metastases can be fully resected (removed surgically). No curative treatment exists for cholangiocarcinoma except surgery. Unfortunately, most patients have advanced stage disease which is inoperable at the time of diagnosis. Patients with cholangiocarcinoma are generally managed - though never cured - with chemotherapy, radiation therapy, and other palliative care measures. These are also used as adjuvant therapies (i.e. post-surgery) in cases where resection has apparently been successful (or nearly so).

In the western hemisphere cholangiocarcinoma is a relatively rare neoplasm that is classified as an adenocarcinoma (a cancer that forms glands or secretes significant amounts of mucins). It has an annual incidence rate of 1-2 cases per 100,000 in the Western world. However, rates of cholangiocarcinoma have been rising worldwide over the past several decades. Furthermore the incidence is higher in Asian countries where it is recognized as a significant problem.

Thus there a huge clinical need for improved therapies to address the lack effective treatment options for patients with biliary cancer.

(*R*)-N4-[3-Chloro-4-(thiazol-2-ylmethoxy)-phenyl]-N6-(4-methyl-4,5,-dihydro-oxazol-2-yl)-quinazoline-4,6-diamine (Varlitinib Example 52 disclosed in WO2005/016346), is a small-molecule pan-HER inhibitor. It has been tested as a monotherapy in phase I clinical trials of gastric cancer patients. 23 patients, who had previously failed on one or more rounds of chemotherapy, and where eligible for trastuzumab, each received 500mg of Varlitinib orally twice daily (BID) as monotherapy for 28 days. Tumour biopsies taken before and after treatment were analysed using immunohistochemistry. Signs of clinical activity included downregulation of signalling pathways responsible for cell proliferation, and a reduction in cell survival and cell proliferation in gastric tumours that were either co-expressing EGFR and HER2 or that were HER2 amplified.

In further unpublished clinical work some of the cholangiocarcinoma patients who had previously had several lines of therapy which had failed at some stage were given Varlitinib monotherapy or Varlitinib in combination with chemotherapy. Varlitinib monotherapy / combination therapy showed a surprising level of efficacy in these patients. In addition Varlitinib appears to be efficacious and able to overcome both primary and secondary resistance in biliary duct cancer.

### SUMMARY OF THE DISCLOSURE

Thus there is disclosed a method of treating a biliary duct cancer by administering a therapeutically effective amount of a compound of formula (I): 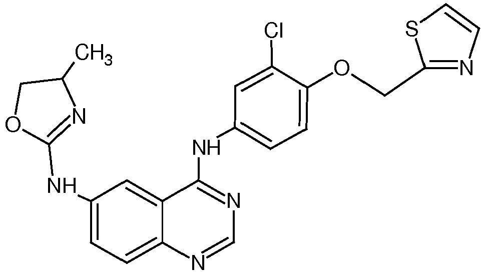 an enantiomer thereof or a pharmaceutically acceptable salt of any one of same.

Thus in one embodiment there is provided a compound of formula (I) for use in the treatment of biliary duct cancer.

In one embodiment the biliary duct cancer is selected from the group consisting of cholangiocarcinoma, gall bladder cancer and a combination thereof.

In one embodiment the disclosure relates to a method of treating a cholangiocarcinoma patient by administering a therapeutically effective amount of a compound of formula (I) an enantiomer thereof or a pharmaceutically acceptable salt of any one of the same.

In one aspect there is provided use of a compound of formula (I) an enantiomer thereof or a pharmaceutically acceptable salt of any one of the same in the treatment of cholangiocarcinoma.

Also provided is a compound of formula (I) an enantiomer thereof or a pharmaceutically acceptable salt of any one of the same for use in the treatment of cholangiocarcinoma.

In one embodiment the cholangiocarcinoma is intrahepatic.

In one embodiment the cholangiocarcinoma is extrahepatic.

In one embodiment the biliary duct cancer is in a location selected from intrahepatic bile ducts, left hepatic duct, right hepatic duct, common hepatic duct, cystic duct, common bile duct, Ampulla of Vater and combinations thereof.

In one embodiment the biliary duct cancer is in an intrahepatic bile duct.

In one embodiment the biliary duct cancer is in a left hepatic duct.

In one embodiment the biliary duct cancer is in a right hepatic duct.

In one embodiment the biliary duct cancer is in a common hepatic duct.

In one embodiment the biliary duct cancer is in a cystic duct.

In one embodiment the biliary duct cancer is in a common bile duct.

In one embodiment the biliary duct cancer is in an Ampulla of Vater.

In one embodiment the biliary duct cancer is a cancer of the Papilla of Vater.

There is also disclosed a method of treating a gallbladder cancer patient by administering a therapeutically effective amount of a compound of formula (I) an enantiomer thereof or a pharmaceutically acceptable salt of any one of the same.

Thus in one aspect there is provided use of a compound of formula (I) an enantiomer thereof or a pharmaceutically acceptable salt of any one of the same in the treatment of gallbladder cancer.

Also provided is a compound of formula (I) an enantiomer thereof or a pharmaceutically acceptable salt of any one of the same for use in the treatment of gallbladder cancer.

In one embodiment the cancer is a metastatic form of a cancer disclosed herein.

In one embodiment the cancer according the present disclosure has not metastasized.

In one embodiment the compound of formula (I) is (*R*)-N4-[3-Chloro-4-(thiazol-2-ylmethoxy]-phenyl]-N6-(4-methyl-4,5,-dihydro-oxazol-2-yl)-quinazoline-4,6-diamine: 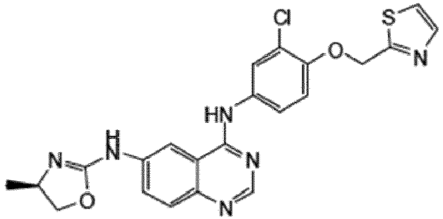 (Varlitinib) or a pharmaceutically acceptable salt thereof or a pro-drug thereof.

In one embodiment (*R*)-N4-[3-Chloro-4-(thiazol-2-ylmethoxy)-phenyl]-N6-(4-methyl-4, 5,-dihydro-oxazol-2-yl)-quinazoline-4,6-diamine is employed/administered as the free base (also referred to herein as Varlitinib).

Varlitinib at an appropriate dose is capable of inhibiting HER1, HER2 and HER4 directly and thought to be capable of inhibiting HER3 indirectly.

In one embodiment the compound of formula (I), such as Varlitinib at least inhibits the activity of HER1 and HER2, HER1 and HER4 or HER2 and HER4.

In one embodiment the compound of formula (I) at least inhibits the activity of HER1, HER2 and HER4, for example directly inhibits the activity of HER1, HER2 and HER4.

In one embodiment the compound of formula (I) inhibits the activity of HER1, HER2, HER3 and HER4, for example directly inhibits the activity of HER1, HER2, and HER4, and indirectly inhibits the activity of HER3.

In one embodiment the compound of formula (I) an enantiomer thereof or a pharmaceutically acceptable salt thereof is employed as a monotherapy, for example first line therapy or second line therapy, such as a first line monotherapy.

In one embodiment the compound of formula (I) an enantiomer thereof or a pharmaceutically acceptable salt thereof is employed in a combination therapy, for example in combination with a chemotherapy and/or a biological therapeutic, in particular as a first line therapy or a second line therapy.

In one embodiment the compound of formula (I), such as Varlitinib, is employed in combination with at least one further HER inhibitor, for example a combination of Varlitinib and Herceptin (trastuzumab) and/or pertuzumab. Surprisingly a combination of Varlitinib and Herceptin showed more therapeutic activity than either entity alone.

In one embodiment the compound of formula (I) such as Varlitinib is employed in combination with ado-trastuzuma-emtansine.

In one embodiment there is disclosed a method of treating biliary duct cancer comprising:
a) administering a therapeutically effective amount of a compound of formula (I): 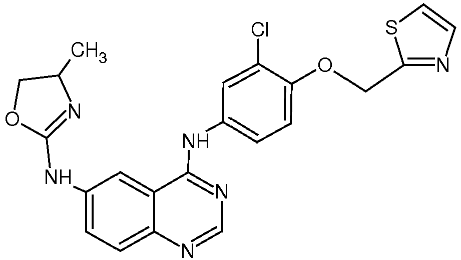 an enantiomer thereof or a pharmaceutically acceptable salt of any one of the same, and
b) administering a chemotherapeutic agent or a combination of chemotherapeutic agents.

In one embodiment the compound of formula (I) an enantiomer thereof or a pharmaceutically acceptable salt thereof is employed as a second line monotherapy.

Surprisingly in a second line monotherapy trial employing Varlitinib in the treatment of cholangiocarcinoma patients showed a significant reduction in CA19-9 levels. CA19-9 is a marker employed in the management of cholangiocarcinoma. In a one patient a 90% reduction was seen in CA19-9 within one month of initiating treatment with Varlitinib.

Thus in one embodiment a patient has a 10, 20, 30, 40, 50, 60, 70, 80 or 90% decrease in CA19-9 level whilst on the therapy according to the present disclosure, wherein the level is decreased relative to the level of CA19-9 before initiation of said therapy.

In one embodiment the compound of formula (I), such as Varlitinib, is employed in a second line therapy together with a chemotherapy agent or chemotherapy regimen, for example gemcitabine, capecitabine, 5-FU, FOLFOX, a platin, such as cisplatin or oxaliplatin, and a combination thereof.

In one embodiment the compound of formula (I), such as Varlitinib, is administered orally.

According to the invention, the compound of formula (I), such as Varlitinib, is administered at a dose in the range 100mg to 500mg on each occasion, in particular 200 mg, 300mg, 400mg or 500mg each dose, such as 400mg, for example administered once or twice daily, such as twice daily.

In one embodiment the compound of formula (I), such as Varlitinib, is administered for a 28 days, referred to herein as a 28 day treatment cycle.

In one embodiment the compound of formula (I) is administered as pharmaceutical formulation comprising one or more pharmaceutically acceptable excipients.

In one embodiment the compound of formula (I) or a formulation comprising the same is administered orally, for example as tablet or capsule.

In one embodiment the target patient population is at least HER1 (EGFR) positive.

In one embodiment the target patient population is EGFR and HER2 positive.

In one embodiment the target patient population is at least HER1 (EGFR) positive.

In one embodiment the target patient population are HER2 amplified.

In one embodiment the patient has over-expression of 2 or more of the HER 1, 2, 3, 4 receptors, for example over expression of HER 1 & 2; 1 & 3; 1 & 4; 2 & 3 2 & 4; 1, 2 & 3; 1, 2 & 4; 2, 3 & 4; or 1, 2, 3 & 4.

In one embodiment the treatment is adjuvant therapy, for example after surgery or after chemotherapy.

In one embodiment the treatment is neoadjuvant therapy, for example before surgery, in particular to shrink the tumour or tumours.

In one embodiment the cancer is a tumour, in particular a solid tumour. In one embodiment the treatment according to the present disclosure is suitable for the treatment of secondary tumours. In one embodiment the cancer is metastatic cancer. In one embodiment the treatment according to the present disclosure is suitable for the treatment of primary cancer and metastases. In one embodiment the treatment according to the present disclosure is suitable for the treatment of secondary cancer and metastases. In one embodiment the treatment according to the present disclosure is suitable for the treatment of primary cancer, secondary cancer and metastases.

In one embodiment the treatment according to the present disclosure is suitable for the treatment of cancerous cells in a lymph node, for a cancer of the present disclosure.

In one embodiment the patient is a human.

### DETAILED DISCLOSURE

Biliary duct cancer (also referred to as biliary cancer) as employed herein refers to cancer which starts in the bile ducts and includes cholangiocarcinoma and gallbladder cancer.

Cholangiocarcinoma as referred to herein is a form of cancer that is composed of mutated epithelial cells (or cells showing characteristics of epithelial differentiation) that originate in the bile ducts which drain bile from the liver into the small intestine, but not including gallbladder cancer.

General guidelines for operability include:
- Absence of lymph node or liver metastases
- Absence of involvement of the portal vein
- Absence of direct invasion of adjacent organs
- Absence of widespread metastatic disease

Gallbladder cancer as employed herein cancer which starts in the gallbladder. The following stages are used for gallbladder cancer:
- Stage 0 (carcinoma *in situ*) : Abnormal cells are found in the inner (mucosal) layer of the gallbladder; these abnormal cells may become cancer and spread into nearby normal tissue,
- Stage I: Cancer has formed and has spread beyond the inner (mucosal) layer to a layer of tissue with blood vessels or to the muscle layer,
- Stage II: Cancer has spread beyond the muscle layer to the connective tissue around the muscle,
- Stage IIIA : Cancer has spread through the thin layers of tissue that cover the gallbladder and/or to the liver and/or to one nearby organ (e.g., stomach, small intestine, colon, pancreas, or bile ducts outside the liver),
- Stage IIIB : Cancer has spread to nearby lymph nodes and beyond the inner layer of the gallbladder to a layer of tissue with blood vessels or to the muscle layer; or beyond the muscle layer to the connective tissue around the muscle; or through the thin layers of tissue that cover the gallbladder and/or to the liver and/or to one nearby organ,
- Stage IVA: Cancer has spread to a main blood vessel of the liver or to 2 or more nearby organs or areas other than the liver. Cancer may have spread to nearby lymph nodes.
- Stage IVB : Cancer has spread to either lymph nodes along large arteries in the abdomen and/or near the lower part of the backbone or to organs or areas far away from the gallbladder.

In one embodiment the treatment of the present disclosure is neo-adjuvant therapy, for example to shrink the tumour/carcinoma before surgery to remove the cancerous tissue or before chemotherapy to improve the chances of success of the latter or to reduce the severity of the treatment required.

In one embodiment the treatment of the present disclosure is adjuvant therapy, for example following surgery to remove the cancerous tissue.

In one embodiment the treatment of the present disclosure is adjuvant therapy, for example following chemotherapy.

In patients where not all the cancerous tissue is removed by surgery then the patient may benefit from adjuvant therapy which is monotherapy employing a compound of formula (I), such as Varlitinib.

In patients where not all the cancerous tissue is removed by surgery then the patient may benefit from combination adjuvant therapy comprising a compound of formula (I) and chemotherapy or radiotherapy.

First line therapy as employed herein is the first therapy employed for the treatment of the cancer and in some instances the first line therapy may be neo-adjuvant therapy, in this context surgery will generally be considered a treatment.

Second line therapy as employed herein is treatment following first line therapy and may be adjuvant therapy. Thus in the context of the present specification second line therapy is simply therapy other than first line therapy and includes, third line therapy, fourth line therapy etc.

Monotherapy as employed herein is wherein the compound of formula (I) an enantiomer thereof and/or a pharmaceutically acceptable salt thereof, is the only active agent being administered to the patient for the treatment of cancer.

Combination therapy as employed herein refers to wherein the compound of formula (I) an enantiomer thereof or a pharmaceutically acceptable salt thereof is employed for the treatment of the cancer in conjunction with one or more further anticancer treatments, for example where the treatment regimens for the two or more active anticancer agents overlap or where the two or more anticancer agents are administered concomitantly.

In one embodiment the combination therapy according to the present disclosure comprises a RON inhibitor, for example as disclosed WO2008/058229, incorporated herein by reference.

In one embodiment the combination therapy comprises a checkpoint inhibitor, such as a CTLA4 inhibitor, a PD-1 inhibitor or a PD-L1 inhibitor, in particular an antibody or binding fragment thereof.

Examples of pharmaceutically acceptable salts include but are not limited to acid addition salts of strong mineral acids such as HCl and HBr salts and addition salts of strong organic acids, such as a methansulfonic acid salt, tosylates, furoates and the like, including di, tri salts thereof, such as ditosylates.

Analysis of patients to profile their cancer, for example to establish if their cancer is EGFR and HER2 positive is known and is routine in the art. Establishing if a cancer is HER2 amplified is also routine in the art.

### Chemotherapeutic Agents

Chemotherapeutic agent and chemotherapy or cytotoxic agent are employed interchangeably herein unless the context indicates otherwise.

Chemotherapy as employed herein is intended to refer to specific antineoplastic chemical agents or drugs that are "selectively" destructive to malignant cells and tissues, for example alkylating agents, antimetabolites including thymidylate synthase inhibitors, anthracyclines, anti-microtubule agents including plant alkaloids, taxanes, topoisomerase inhibitors, parp inhibitors and other antitumour agents. Selectively in this context is used loosely because of course many of these agents have serious side effects.

The preferred dose may be chosen by the practitioner, based on the nature of the cancer being treated.

Examples of alkylating agents, which may be employed in the method of the present disclosure include an alkylating agent, nitrogen mustards, nitrosoureas, tetrazines, aziridines, platins and derivatives, and non-classical alkylating agents.

Examples of platinum containing chemotherapeutic agents (also referred to as platins), include cisplatin, carboplatin, oxaliplatin, satraplatin, picoplatin, nedaplatin, triplatin and lipoplatin (a liposomal version of cisplatin), in particular cisplatin, carboplatin and oxaliplatin.

The dose for cisplatin ranges from about 20 to about 270 mg/m² depending on the exact cancer. Often the dose is in the range about 70 to about 100mg/m².

Nitrogen mustards include mechlorethamine, cyclophosphamide, melphalan, chlorambucil, ifosfamide and busulfan.

Nitrosoureas include N-Nitroso-N-methylurea (MNU), carmustine (BCNU), lomustine (CCNU) and semustine (MeCCNU), fotemustine and streptozotocin. Tetrazines include dacarbazine, mitozolomide and temozolomide.

Aziridines include thiotepa, mytomycin and diaziquone (AZQ).

Examples of antimetabolites, which may be employed in the method of the present disclosure, include anti-folates (for example methotrexate and pemetrexed), purine analogues (for example thiopurines, such as azathiopurine, mercaptopurine, thiopurine, fludarabine (including the phosphate form), pentostatin and cladribine), pyrimidine analogues (for example fluoropyrimidines, such as 5-fluorouracil (5-FU) and prodrugs thereof such as capecitabine [Xeloda®]), floxuridine, gemcitabine, cytarabine, decitabine, raltitrexed (tomudex) hydrochloride, cladribine and 6-azauracil.

Examples of anthracyclines, which may be employed in the method of the present disclosure, include daunorubicin (Daunomycin), daunorubicin (liposomal), doxorubicin (Adriamycin), doxorubicin (liposomal), epirubicin, idarubicin, valrubicin currently are used only to treat bladder cancer and mitoxantrone an anthracycline analog, in particular doxorubicin.

Examples of anti-microtubule agents, which may be employed in the method of the present disclosure, include include vinca alkaloids and taxanes.

Vinca alkaloids include completely natural chemicals for example vincristine and vinblastine and also semi-synthetic vinca alkaloids, for example vinorelbine, vindesine, and vinflunine

Taxanes include paclitaxel, docetaxel, abraxane, carbazitaxel and derivatives of thereof. Derivatives of taxanes as employed herein includes reformulations of taxanes like taxol, for example in a micelluar formulaitons, derivatives also include chemical derivatives wherein synthetic chemistry is employed to modify a starting material which is a taxane.

Topoisomerase inhibitors, which may be employed in a method of the present disclosure include type I topoisomerase inhibitors, type II topoisomerase inhibitors and type II topoisomerase poisons. Type I inhibitors include topotecan, irinotecan, indotecan and indimitecan. Type II inhibitors include genistein and ICRF 193 which has the following structure:

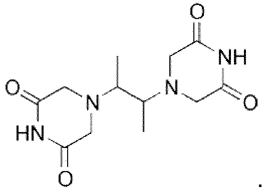

Type II poisons include amsacrine, etoposide, etoposide phosphate, teniposide and doxorubicin and fluoroquinolones.

In one embodiment the chemotherapeutic is a PARP inhibitor.

In one embodiment a combination of chemotherapeutic agents employed is, for example a platin and 5-FU or a prodrug thereof, for example cisplatin or oxaplatin and capecitabine or gemcitabine, such as FOLFOX.

In one embodiment the chemotherapy comprises a combination of chemotherapy agents, in particular cytotoxic chemotherapeutic agents.

In one embodiment the chemotherapy combination comprises a platin, such as cisplatin and fluorouracil or capecitabine.

In one embodiment the chemotherapy combination is capecitabine and oxaliplatin (XELOX).

In one embodiment the chemotherapy is a combination of folinic acid and 5-FU, optionally in combination with oxaliplatin (FOLFOX).

In one embodiment the chemotherapy is a combination of folinic acid, 5-FU and irinotecan (FOLFIRI), optionally in combination with oxaliplatin (FOLFIRINOX). The regimen, for example includes: irinotecan (180 mg/m² IV over 90 minutes) concurrently with folinic acid (400 mg/m² [or 2 x 250 mg/m²] IV over 120 minutes); followed by fluorouracil (400-500 mg/m² IV bolus) then fluorouracil (2400-3000 mg/m² intravenous infusion over 46 hours). This cycle is typically repeated every two weeks. The dosages shown above may vary from cycle to cycle.

In one embodiment the chemotherapy combination employs a microtubule inhibitor, for example vincristine sulphate, epothilone A, N-[2-[(4-Hydroxyphenyl)amino]-3-pyridinyl]-4-methoxybenzenesulfonamide (ABT-751), a taxol derived chemotherapeutic agent, for example paclitaxel, abraxane, or docetaxel or a combination thereof.

In one embodiment the chemotherapy combination employs an mTor inhibitor. Examples of mTor inhibitors include: everolimus (RAD001), WYE-354, KU-0063794, papamycin (Sirolimus), Temsirolimus, Deforolimus(MK-8669), AZD8055 and BEZ235(NVP-BEZ235).

In one embodiment the chemotherapy combination employs a MEK inhibitor. Examples of MEK inhibitors include: AS703026, CI-1040 (PD184352), AZD6244 (Selumetinib), PD318088, PD0325901, AZD8330, PD98059, U0126-EtOH, BIX 02189 or BIX 02188.

In one embodiment the chemotherapy combination employs an AKT inhibitor. Examples of AKT inhibitors include: MK-2206 and AT7867.

In one embodiment the combination employs an aurora kinase inhibitor. Examples of aurora kinase inhibitors include: Aurora A Inhibitor I, VX-680, AZD1152-HQPA (Barasertib), SNS-314 Mesylate, PHA-680632, ZM-447439, CCT129202 and Hesperadin.

In one embodiment the chemotherapy combination employs a p38 inhibitor, for example as disclosed in WO2010/038086, such as *N*-[4-({4-[3-(3-*tert*-Butyl-1-*p*-tolyl-1*H-*pyrazol-5-yl)ureido]naphthalen-1-yloxy}methyl)pyridin-2-yl]-2-methoxyacetamide.

In one embodiment the combination employs a Bcl-2 inhibitor. Examples of Bcl-2 inhibitors include: obatoclax mesylate, ABT-737, ABT-263(navitoclax) and TW-37.

In one embodiment the chemotherapy combination comprises an antimetabolite such as capecitabine (xeloda), fludarabine phosphate, fludarabine (fludara), decitabine, raltitrexed (tomudex), gemcitabine hydrochloride and/or cladribine.

In one embodiment the chemotherapy combination comprises ganciclovir, which may assist in controlling immune responses and/or tumour vasculation.

In one embodiment after combination therapy a monotherapy comprising a compound of formula (I), such as Varlitinib (as defined herein including doses described above) is employed, for example a maintenance therapy.

"Comprising" in the context of the present specification is intended to mean "including". Where technically appropriate, embodiments of the invention may be combined.

Embodiments are described herein as comprising certain features/elements. The disclosure also extends to separate embodiments consisting or consisting essentially of said features/elements.

Any embodiments specifically and explicitly recited herein may form the basis of a disclaimer either alone or in combination with one or more further embodiments.

The invention will now be described with reference to the following examples.

### BRIEF SUMMARY OF THE FIGURES

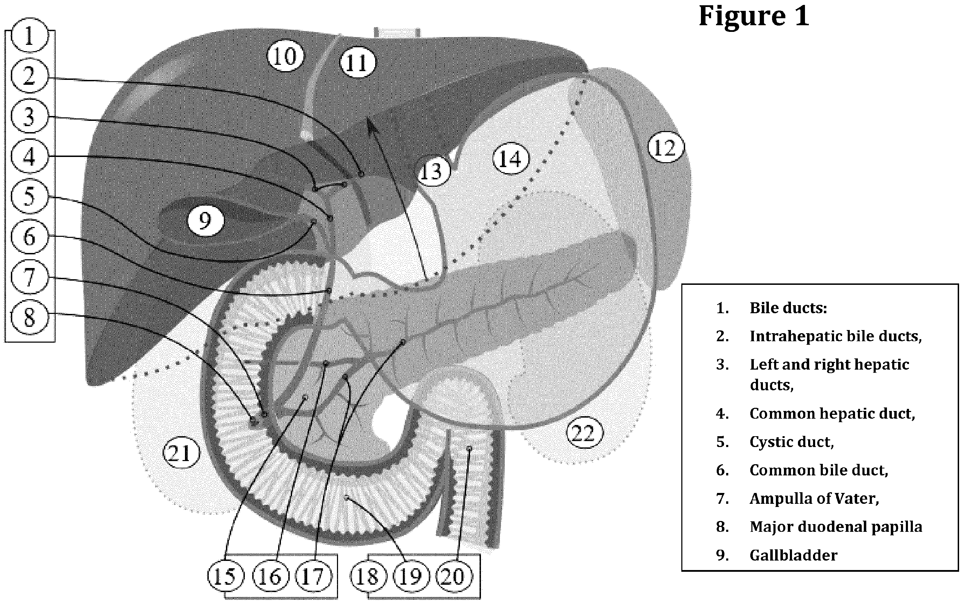

- **Figure 1**: Shows the position of bile ducts in the body
- **Figure 2**: Shows data for a cholangiocarcinoma patient receiving 400mg of Varlitinib monotherapy bi-daily
- **Figure 3**: Shows data for a cholangiocarcinoma patient receiving 400mg Varlitinib bi-daily in combination with chemotherapy.
- **Figure 4**: Shows data for a 56 year old male cholangiocarcinoma patient treated with Varlitinib 400mg bi-daily and cisplatin/capecitabine chemotherapy
- **Figure 5**: Shows data for a 60 year old male cholangiocarcinoma patient treated with Varlitinib 500mg bi-daily and cisplatin/capecitabine chemotherapy

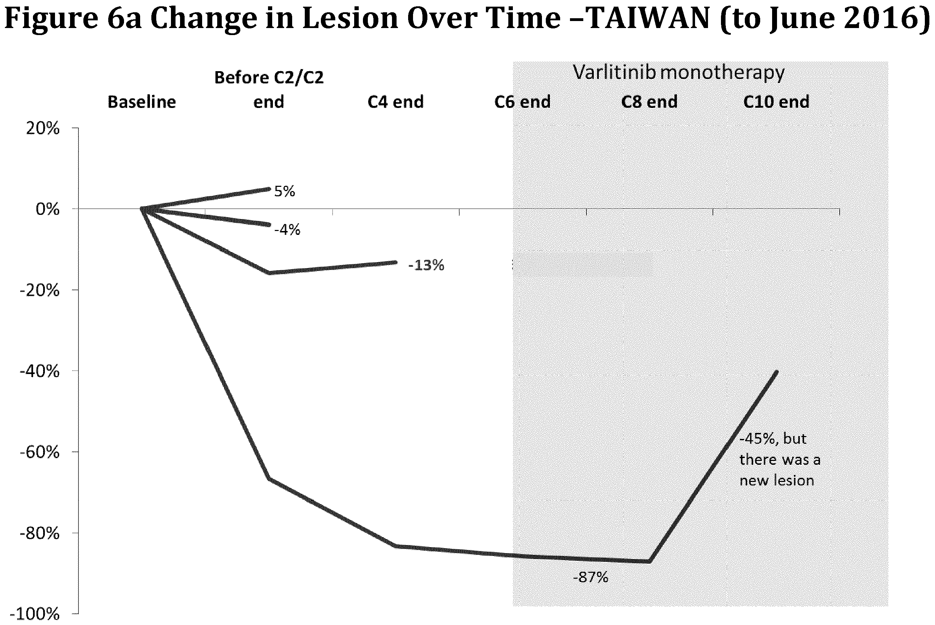

- **Figure 6a**: Shows change in a lesion of over time for individual cholangiocarcinoma patients receiving between 300 and 500mg of Varlitinib bi-daily and chemotherapy in a Taiwanese clinical trial

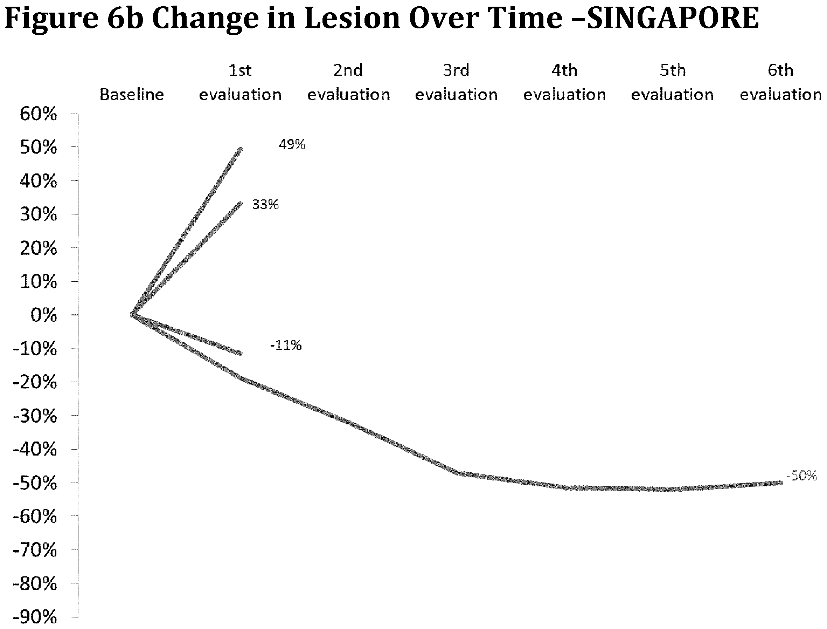

- **Figure 6b**: Shows change in a lesion of over time for individual cholangiocarcinoma patients receiving between 300 and 500mg of Varlitinib bi-daily and chemotherapy in a Singapore clinical trial

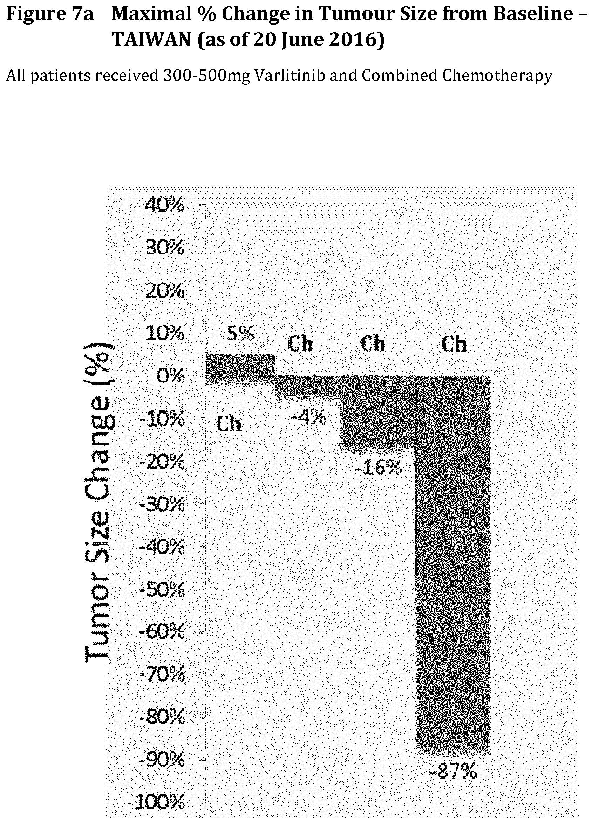

- **Figure 7a**: Shows the maximal % change in tumour size from baseline in individual cholangiocarcinoma patients receiving between 300-500mg of Varlitinib bi-daily and chemotherapy in Taiwan clinical trial

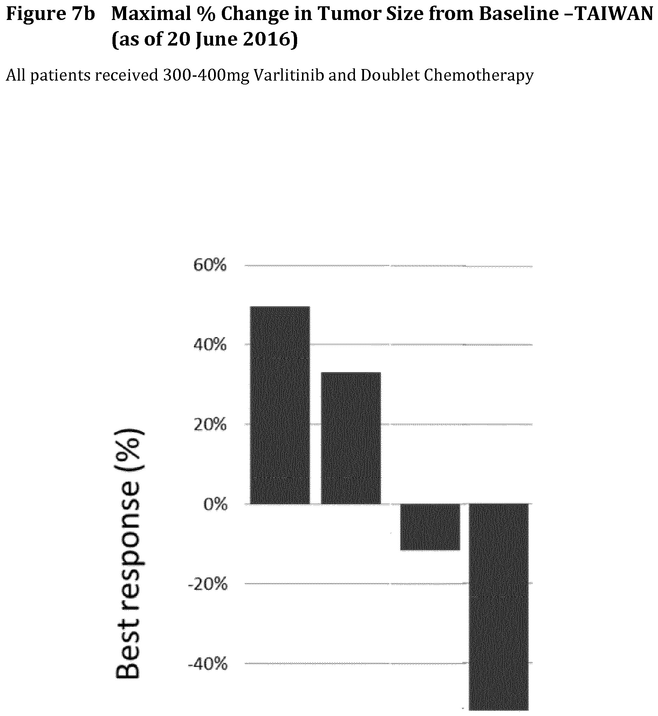

- **Figure 7b**: Shows the maximal % change in tumour size from baseline in individual cholangiocarcinoma patients receiving between 300-500mg of Varlitinib bi-daily and chemotherapy in Taiwan clinical trial

### EXAMPLES

### Example 1 Varlitinib 400mg bi-daily Monotherapy

A 45 year-old male stage IV cholangiocarcinoma EGFR positive (3+) cancer patient had progressive disease following:
- first line treatment with gemcitabine (partial remission), and
- second line treatment with cisplatin and 5-FU.

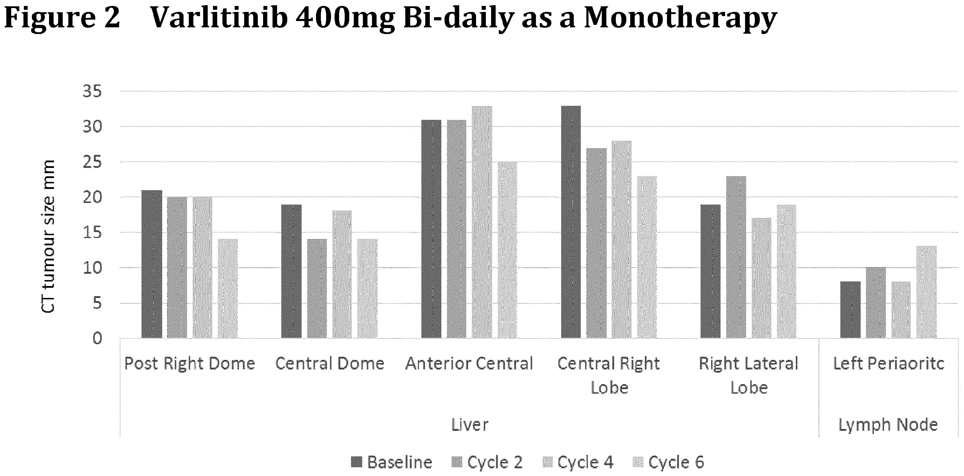

The results are shown in Figure 2. After treatment cycle 6 with Varlitinib 400mg bi-daily the liver tumours decreased in size up to 23% and the tumour marker CA 19-9 fell from ∼900 U/ml to -250 U/ml.

### Example 2 Varlitinib 400mg bi-daily Monotherapy

A 58 year-old, male, stage IV, extra-hepatic cholangiocarcinoma, prior treatment:
- Whipple
- Radiotherapy
- Gemzar/ cisplatin for 6 months

For the first 6 cycles, the patient received Varlitinib 400mg BID continuously with cisplatin (80mg/m² every 3 weeks) and capecitabine (1000mg/m2 BID, 2 weeks on, 1 week off). Image scan at the end of cycle 6 showed 85.77% reduction in tumour size. After cycle 6, the patient received varlitinib monotherapy and tumor scan at the end of cycle 8 showed 87% reduction.

This patient showed partial remission for 24 weeks. However, at the end of cycle 10, image scan showed tumour enlarged to the extent that met criteria for disease progression based on RECIST (>20% increase against nadir), so the patient was withdrawn from this study. In summary, the patient received varlitinib with chemo for 6 cycles and varlitinib monotherapy for another 4 cycles (3 weeks per cycle).

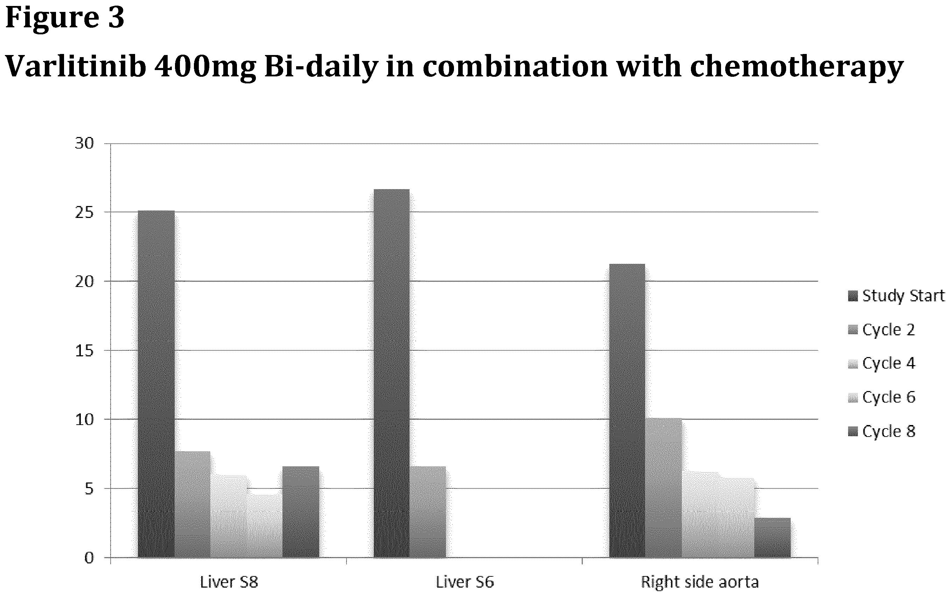

The results are shown in Figure 3.

### Example 3 Treatment of Cholangiocarcinoma with Varlitinib 400mg bi-daily orally and cisplatin/capecitabine combination chemotherapy

A 56 year-old male with stage IV cholangiocarcinoma (3 lesions) had progressive disease following treatment with:
- radiotherapy, and
- gemcitabine (Gemzar®) and cisplatin -6 months.

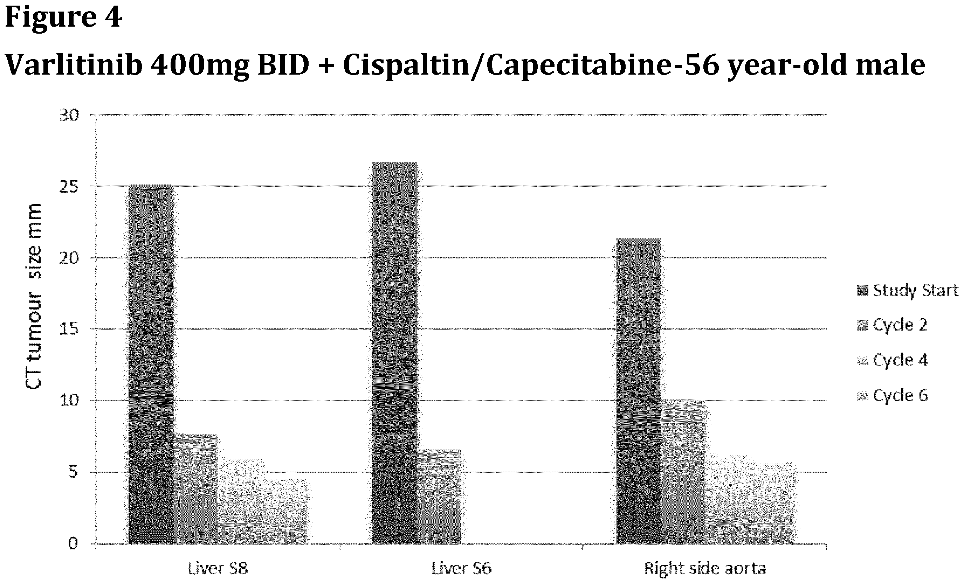

After treatment cycle 6 with Varlitinib 400mg bi-daily, cisplatin and capecitabine the patient showed an 85.77% response, see Figure 4. No dose limiting toxicity was observed in the first two treatment cycles. Varlitinib was well tolerated. The cisplatin and capecitabine regime was cisplatin 80mg/m² IV infusion and capecitabine 1000mg/m² orally twice daily for 14 days every 3 weeks.

### Example 4 Treatment of Cholangiocarcinoma with Varlitinib 500mg bi-daily orally and cisplatin/capecitabine combination chemotherapy

A 60 year-old male with stage IV cholangiocarcinoma had progressive disease following treatment with radiotherapy and bi-weekly 5-FU.

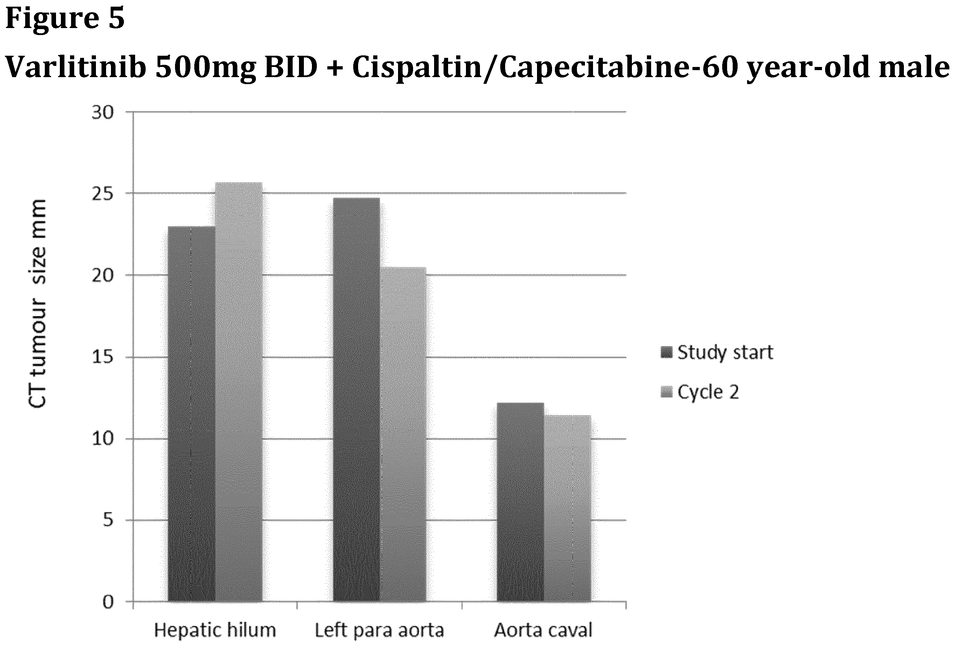

After treatment cycle 2 with Varlitinib orally 500mg bi-daily, cisplatin and capecitabine the patient showed a 4% response, see Figure 5. The cisplatin and capecitabine regime was cisplatin 80mg/m² IV infusion and capecitabine 1000mg/m² orally twice daily for 14 days every 3 weeks.

### Example 5 Treatment of Stage IV Cholangiocarcinoma and metastatic lymphadenopathy with Varlitinib 400mg bi-daily orally and cisplatin/5FU combination chemotherapy

A 49 year-old male was diagnosed with stage IV cholangiocarcinoma and metastatic lymphadenopathy in January 2016. No prior surgery or treatment was given before initiating first line treatment of 300mg BID Varlitinib combined with Cisplating/5-FU, 28-day treatment cycle.

Date of the first response 5 March 2016, tumor C2 was reduced by 16%. Last response tumor C4 was reduced by 13% (17 May 2016). Patient current disease status is stable disease.

### Example 6 Treatment of the Intrahepatic Bile Duct Cholangiocarcinoma with 300mg Valitinib and FOLFOX

A 51 year old female diagnosed in 2013 with intrahepatic bile duct cholangiocarcinoma, had received surgery in the form of left hemihepatectomy in 15 July 2013. Prior therapies were gemcitabine and cisplatin (in the period 14 August 2013 to 8 January 2014) and repeated between 13 May 2015 and 1 Jul 2015. With this treatment the status was progressive disease. The patient was given 9 cycles of a combination of Varlitinib 400mg reduced to 300mg BID and FOLFOX. This was followed with 7 cycles of Varlitinib monotherapy. The patient status is partial response with a reduction is tumor size of 50%.

### Example 7 Treatment of Stage IV Cholangiocarcinoma with Varlitinib 400mg bi-daily orally and cisplatin/5FU combination chemotherapy

A 69 year-old female with stage IV cholangiocarcinoma newly diagnosed in 2014 received surgery (percutaneous transhepatic cholangial drainage and transcatheter anterior embolization), followed by adjvant treatment of Varlitinib 400mg BID combined with cisplatin s/5-FU 28-day cycle. After cycle 6 (14Jul2015), only 400mg BID Varlitinib was taken. Patient completed 10 cycles of monotherapy. At Cycle 11 Day 1, dose was reduced to 300mg BID alternate days. First response was observed in cycle 2 of treatment 17 March 2015. Progressive disease observed in cycle 16 of monotherapy 12 April 2016. This patient demonstrated remarkable disease control on varlitinib monotheerapy after 6 cycles of varlitinib in combination with chemotherapy.

### Example 8 Treatment of Stage IV Intrahepatic Cholangiocarcinoma with Varlitinib 400mg bi-daily orally and FOLFOX

A 51 year-old female who had a medical history of meningioma post excision with left craniotomy was diagnosed with intrahepatic cholangiocarcinoma, stage IV with metastasis to portacaval lymph nodes and liver in Aug 2014. She received left hemihepatectomy followed by 6 cycles of adjuvant gemcitabine and cisplatin. The patient's disease progressed in May 2015 and she received 1st line gemcitabine and cisplatin in metastatic setting, to which he did not respond. After disease progression on gemcitabine and cisplatin, she was enrolled into the ASLAN001-002SG study in August 2015 and received Varlitinib 400 mg BID in combination with mFOLFOX6 (2 weeks per cycle). To date (22nd Aug 2016), the patient has completed 9 cycles of Varlitinib and mFOLFOX6, as well as, 9 cycles of varlitinib monotherapy and the latest tumor assessment completed after cycle 18 continues to show partial response with 53% reduction in tumor size as the best response from baseline.

### Example 9 Treatment of Stage IV Intrahepatic Cholangiocarcinoma and multiple lymphadenopathies with Varlitinib 300mg bi-daily orally and cisplatin/5FU combination chemotherapy 28 day cycle

**A** 64 year old female diagnosed with intrahepatic cholangiocarcinoma and multiple lymphadenopathies in May 2016 received Varlitinib combination therapy as the first line treatment.

The patient was given 300 mg BID Varlitinib combined with Cis/5-FU, 28-day cycle. The current status is the clinical trial is on-going.

## Claims

1. A dose in the range 100mg to 500mg of a compound of formula (I): 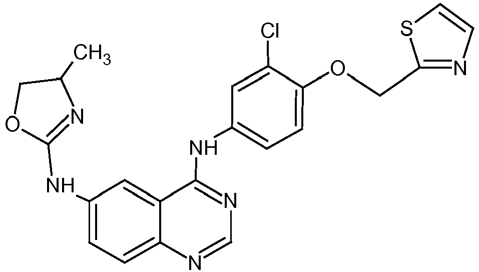 an enantiomer thereof or a pharmaceutically acceptable salt of any one of the same for use in the treatment of biliary duct cancer.

2. A compound for use according to claim1, for treating a cholangiocarcinoma patient by administering a therapeutically effective amount of a compound of formula (I): 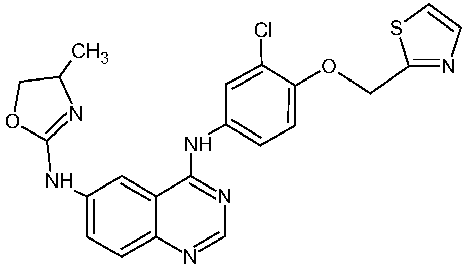 an enantiomer thereof and/or pharmaceutically acceptable salts thereof.

3. A compound for use according to claim 1, wherein the biliary duct cancer is gallbladder cancer.

4. A compound for use according to claim 2, wherein the cholangiocarcinoma is located in an intrahepatic bile duct, left hepatic duct, right hepatic duct, common hepatic duct, cystic duct, common bile duct, Ampulla of Vater or a combination thereof.

5. A compound for use according to any one of claims 1, 2, or 4, wherein the cholangiocarcinoma is intrahepatic.

6. A compound for use according to any one of claims 1, 2 4 or 5, wherein the cholangiocarcinoma is extrahepatic.

7. A compound for use according to any one of claims 1 to 6, wherein the compound of formula (I) is Varlitinib: 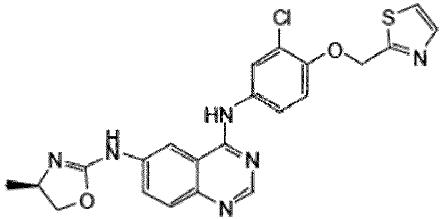 or a pharmaceutically acceptable salt thereof.

8. A compound for use according to any one of claims 1 to 7, wherein the compound of formula (I) is provided as the free base.

9. A compound for use according to any one of claims 1 to 8, wherein the compound of formula (I) is administered as a pharmaceutical formulation.

10. A compound for use according to any one of claims 1 to 9, wherein the compound of formula (I) or a pharmaceutical formulation comprising same is administered orally.

11. A compound for use according to any one of claims 1 to 10 wherein the compound of formula (I) or a pharmaceutical formulation comprising the same is administered bi-daily.

12. A compound for use according to any one of claims 1 to 11, wherein each dose is 400mg.

13. A compound for use according to any one of claims 1 to 12, wherein the compound of formula (I) or formulation comprising the same is employed as a monotherapy.

14. A compound for use according to any one of claims 1 to 12, wherein the compound of formula (I) is employed in a combination.

15. A compound for use according to claim 14, wherein the combination therapy comprises Herceptin and/or pertuzumab.

16. A compound for use according to claim 16, wherein the combination therapy comprise adotrastuzumab.

17. A compound for use according to any one of claims 14 to 16, wherein the combination therapy comprises a chemotherapeutic agent.

18. A compound for use according to claim 17, wherein the chemotherapeutic agent is independently from the group comprising a platin, gemcitabine, capecitabine, 5-FU, FOLFOX, FOLFIRI and FOLFIRINOX.

## Patentansprüche

1. Dosis in dem Bereich von 100 mg bis 500 mg einer Verbindung der Formel (I): 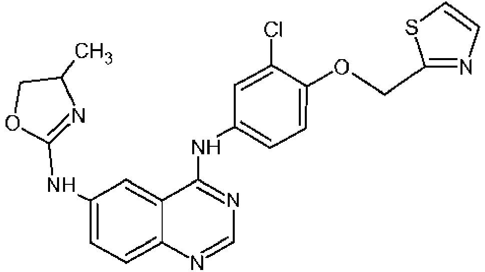 ein Enantiomer davon oder ein pharmazeutisch unbedenkliches Salz eines beliebigen desselben zur Verwendung bei der Behandlung von Gallengangkrebs.

2. Verbindung zur Verwendung nach Anspruch 1 zum Behandeln eines Cholangiokarzinom-Patienten durch Verabreichen einer therapeutisch wirksamen Menge einer Verbindung der Formel (I): 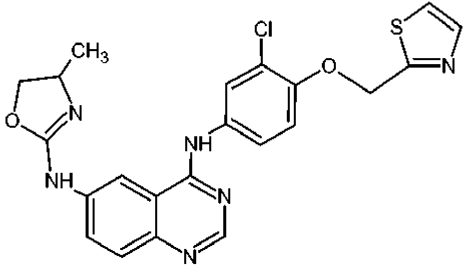 ein Enantiomer davon und/oder pharmazeutisch unbedenkliche Salze davon.

3. Verbindung zur Verwendung nach Anspruch 1, wobei der Gallengangkrebs Gallenblasenkrebs ist.

4. Verbindung zur Verwendung nach Anspruch 2, wobei sich das Cholangiokarzinom in einem intrahepatischen Gallengang, linken Lebergang, rechten Lebergang, gemeinsamen Lebergang, Zystikus, gemeinsamen Gallengang, Vaterschen Ampulle oder einer Kombination davon befindet.

5. Verbindung zur Verwendung nach einem der Ansprüche 1, 2 oder 4, wobei das Cholangiokarzinom intrahepatisch ist.

6. Verbindung zur Verwendung nach einem der Ansprüche 1, 2, 4 oder 5, wobei das Cholangiokarzinom extrahepatisch ist.

7. Verbindung zur Verwendung nach einem der Ansprüche 1 bis 6, wobei die Verbindung der Formel (I) Varlitinib ist: 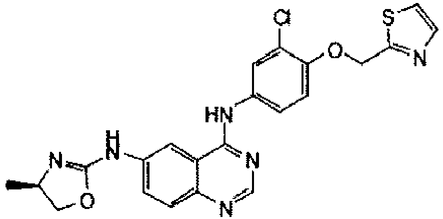 oder ein pharmazeutisch unbedenkliches Salz davon.

8. Verbindung zur Verwendung nach einem der Ansprüche 1 bis 7, wobei die Verbindung der Formel (I) als freie Base bereitgestellt wird.

9. Verbindung zur Verwendung nach einem der Ansprüche 1 bis 8, wobei die Verbindung der Formel (I) als eine pharmazeutische Formulierung verabreicht wird.

10. Verbindung zur Verwendung nach einem der Ansprüche 1 bis 9, wobei die Verbindung der Formel (I) oder eine dieselbe umfassende pharmazeutische Formulierung oral verabreicht wird.

11. Verbindung zur Verwendung nach einem der Ansprüche 1 bis 10, wobei die Verbindung der Formel (I) oder eine dieselbe umfassende pharmazeutische Formulierung zweimal täglich verabreicht wird.

12. Verbindung zur Verwendung nach einem der Ansprüche 1 bis 11, wobei jede Dosis 400 mg beträgt.

13. Verbindung zur Verwendung nach einem der Ansprüche 1 bis 12, wobei die Verbindung der Formel (I) oder eine dieselbe umfassende Formulierung als eine Monotherapie eingesetzt wird.

14. Verbindung zur Verwendung nach einem der Ansprüche 1 bis 12, wobei die Verbindung der Formel (I) in einer Kombination eingesetzt wird.

15. Verbindung zur Verwendung nach Anspruch 14, wobei die Kombinationstherapie Herceptin und/oder Pertuzumab umfasst.

16. Verbindung zur Verwendung nach Anspruch 16, wobei die Kombinationstherapie Ado-Trastuzumab umfasst.

17. Verbindung zur Verwendung nach einem der Ansprüche 14 bis 16, wobei die Kombinationstherapie ein chemotherapeutisches Mittel umfasst.

18. Verbindung zur Verwendung nach Anspruch 17, wobei das chemotherapeutische Mittel unabhängig von der Gruppe ist, die ein Platin, Gemcitabin, Capecitabin, 5-FU, FOLFOX, FOLFIRI und FOLFIRINOX umfasst.

## Revendications

1. Dose dans la plage de 100 mg à 500 mg d'un composé de formule (I) : 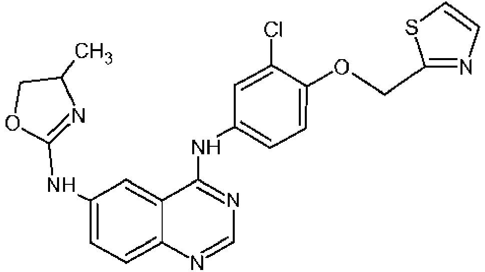 un énantiomère de celui-ci ou un sel pharmaceutiquement acceptable de l'un quelconque de ceux-ci pour une utilisation dans le traitement du cancer des voies biliaires.

2. Composé destiné à être utilisé selon la revendication 1, pour traiter un patient atteint de cholangiocarcinome en administrant une quantité thérapeutiquement efficace d'un composé de formule (I) : 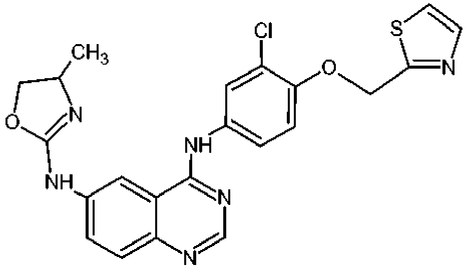 un énantiomère de celui-ci et/ou des sels pharmaceutiquement acceptables de celui-ci.

3. Composé destiné à être utilisé selon la revendication 1, dans lequel le cancer des voies biliaires est un cancer de la vésicule biliaire.

4. Composé destiné à être utilisé selon la revendication 2, dans lequel le cholangiocarcinome est situé dans un canal biliaire intrahépatique, un canal hépatique gauche, un canal hépatique droit, un canal hépatique commun, un canal cystique, un canal cholédoque, une ampoule de Vater ou une combinaison de ceux-ci.

5. Composé destiné à être utilisé selon l'une quelconque des revendications 1, 2 ou 4, dans lequel le cholangiocarcinome est intrahépatique.

6. Composé destiné à être utilisé selon l'une quelconque des revendications 1, 2, 4 ou 5, dans lequel le cholangiocarcinome est extrahépatique.

7. Composé destiné à être utilisé selon l'une quelconque des revendications 1 à 6, dans lequel le composé de formule (I) est du Varlitinib : 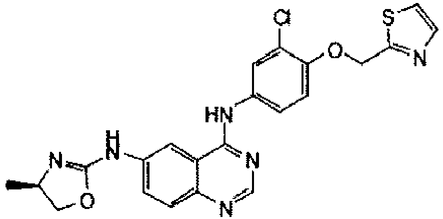 ou un sel pharmaceutiquement acceptable de celui-ci.

8. Composé destiné à être utilisé selon l'une quelconque des revendications 1 ou 7, dans lequel le composé de formule (I) est fourni en tant que base libre.

9. Composé destiné à être utilisé selon l'une quelconque des revendications 1 à 8, dans lequel le composé de formule (I) est administré sous forme de formulation pharmaceutique.

10. Composé destiné à être utilisé selon l'une quelconque des revendications 1 à 9, dans lequel le composé de formule (I) ou une formulation pharmaceutique comprenant celui-ci est administré(e) par voie orale.

11. Composé destiné à être utilisé selon l'une quelconque des revendications 1 à 10, dans lequel le composé de formule (I) ou une formulation pharmaceutique comprenant celui-ci est administré(e) deux fois par jour.

12. Composé destiné à être utilisé selon l'une quelconque des revendications 1 à 11, dans lequel chaque dose est de 400 mg.

13. Composé destiné à être utilisé selon l'une quelconque des revendications 1 à 12, dans lequel le composé de formule (I) ou la formulation comprenant celui-ci est employé(e) en tant que monothérapie.

14. Composé destiné à être utilisé selon l'une quelconque des revendications 1 à 12, dans lequel composé de formule (I) est employé dans une combinaison.

15. Composé destiné à être utilisé selon la revendication 14, dans lequel la polythérapie comprend de l'Herceptin et/ou du pertuzumab.

16. Composé destiné à être utilisé selon la revendication 16, dans lequel la thérapie combinée comprend de l'ado-trastuzumab.

17. Composé destiné à être utilisé selon l'une quelconque des revendications 14 à 16, dans lequel la polythérapie comprend un agent chimiothérapeutique.

18. Composé destiné à être utilisé selon la revendication 17, dans lequel l'agent chimiothérapeutique est indépendamment du groupe comprenant une platine, la gemcitabine, la capécitabine, le 5-FU, le FOLFOX, le FOLFIRI et le FOLFIRINOX.
